# EUROPEAN PATENT SPECIFICATION

(11) **EP 4 464 327 B1**
(45) Date of publication and mention of the grant of the patent: **11.02.2026**
(21) Application number: 24172251.1
(22) Date of filing: 24.04.2024
(51) Int. Cl.: A61K 36/21, A61K 36/534, A61K 31/122, A61P 3/00, A61P 3/06, A23L 33/105

(54) **POWDERED COMPOSITION OF MINT OIL EXTRACT AND AMARANTH SEEDS FLOUR**
ZUSAMMENSETZUNG AUS MINZÖLEXTRAKT UND AMARANTHSAMENMEHL IN PULVERFORM
COMPOSITION EN POUDRE COMPRENANT D' EXTRAIT DE L' HUILE DE MENTHE ET DE LA FARINE DE GRAINES D' AMARANTHE

(30) Priority: 17.05.2023 IT 202300009960
(43) Date of publication of application: 20.11.2024
(73) Proprietor: Gricar Chemical S.r.l., 20861 Brugherio MB (IT)
(72) Inventor: Carenzi, Gian Marco, 20861 Brugherio MB (IT); Contini, Marisa, 20861 Brugherio MB (IT)
(74) Representative: Perani & Partners S.p.A.

(56) References cited:
- RU-C1- 2 232 590
- ASGHARI BEHVAR ET AL: "Amylase, glucosidase, tyrosinase, and cholinesterases inhibitory, antioxidant effects, and GC-MS analysis of wild mint (Mentha longifoliavar.calliantha) essential oil: A natural remedy", EUROPEAN JOURNAL OF INTEGRATIVE MEDICINE, ELSEVIER, AMSTERDAM, NL, vol. 22, 4 August 2018 (2018-08-04), pages 44 - 49, XP085479217, ISSN: 1876-3820, DOI: 10.1016/J.EUJIM.2018.08.004
- MIMICA-DUKIC N ET AL: "Mentha L. Species (Lamiaceae) as Promising Sources of Bioactive Secondary Metabolites", CURRENT PHARMACEUTICAL DESIGN, BENTHAM SCIENCE PUBLISHERS, NL, vol. 14, no. 29, 1 October 2008 (2008-10-01), pages 3141 - 3150, XP009159465, ISSN: 1381-6128, [retrieved on 20081001], DOI: 10.2174/138161208786404245
- RODR?GUEZ MARIELA ET AL: "Polyphenols in amaranth (A. manteggazianus) flour and protein isolate: Interaction with other components and effect of the gastrointestinal digestion", FOOD RESEARCH INTERNATIONAL, ELSEVIER, AMSTERDAM, NL, vol. 137, 21 July 2020 (2020-07-21), XP086360253, ISSN: 0963-9969, [retrieved on 20200721], DOI: 10.1016/J.FOODRES.2020.109524
- ASSAD REZWANA ET AL: "Biology of Amaranths", BOTANICAL REVIEW, NEW YORK BOTANICAL GARDEN, BRONX, NY, US, vol. 83, no. 4, 26 October 2017 (2017-10-26), pages 382 - 436, XP036373525, ISSN: 0006-8101, [retrieved on 20171026], DOI: 10.1007/S12229-017-9194-1
- CONSUELO D?AZ-MAROTO M ET AL: "Volatile composition and olfactory profile of pennyroyal (Mentha pulegium L.) plants", FLAVOUR AND FRAGRANCE JOURNAL, WILEY, NEW YORK, NY, GB, vol. 22, no. 2, 8 December 2006 (2006-12-08), pages 114 - 118, XP071708167, ISSN: 0882-5734, DOI: 10.1002/FFJ.1766

## Description

### FIELD OF THE INVENTION

The field of the present invention relates to a powder composition, effective in treating the metabolic syndrome, comprising two actives, the Mint oil extract and the Amaranth seeds flour, the second one serving as a carrier of the first.

### BACKGROUND ART

The species of the genus *Mentha* contain many interesting active principles, to which different effects are attributed (e.g., antioxidant, antibacterial, antifungal). Some of these species contain terpenes, such as piperitone oxide, whose benefits are turning out to be surprising.

Special attention is paid to wild Mint, whose botanical name is *Mentha spicata subsp. longifolia L..* This grows spontaneously and is widely spread in Europe, Asia and non-tropical areas of Africa. In Italy, it can be found in many regions, especially in the Alpine part, in the range from 900 to 2000 m.

It is a plant traditionally used for treating several diseases, among which gastrointestinal disorders, respiratory disorders, infectious diseases, inflammatory diseases, as well as menstrual disorders. In modern age, its several pharmacological activities, such as antiparasitic, antimicrobial, antinociceptive, anti-inflammatory, antioxidant, keratoprotective, hepatoprotective, anti-diarrheal, and spasmolytic effects, have been confirmed. The plant has shown therapeutic benefits in irritable bowel syndrome, amenorrhea and oligomenorrhea, and in diseases associated with oxidative stress as well.

It contains a wide range of natural components, such as flavonoids, phenolic acids, cinnamates, ceramides, sesquiterpenes, terpenes and terpenoids, which are believed to be responsible for its pharmacological effects.

Beyond the pharmacological effects, the wild Mint can also be useful as an active ingredient of food supplements, also referred to as "nutraceuticals", which aim instead to promote health and, in some cases, to prevent diseases. In recent years, many studies have been conducted in order to assess the effectiveness and safety of nutraceuticals; however, these factors are difficult to address because of the complex chemical compositions and the multiple modes of action. Therefore, the study of nutraceutical ingredients poses several challenges to the pharmaceutical chemistry field, some of which being related to the extraction and chemical characterization, some others to the *in vitro* and *in vivo* bioactivity assessment, and still others to the bioavailability and the interaction of these natural mixtures with organs and microbiota.

Nowadays, increasing attention is paid to possible preventive/alternative modes to avoid the onset of the disease. Lifestyle changes and eating habits are taking over the conventional pharmaceutical approach, especially in preventing chronic conditions, such as, for example, the metabolic syndrome, of which hypercholesterolemia is a component, and which is related to incorrect lifestyle and eating habits. The nutraceutical approach to mild hypercholesterolemia proves, therefore, to be important and one of the major targets of the studies on nutraceuticals.

Ashgari Behvar et al, EUROPEAN JOURNAL OF INTEGRATIVE MEDICINE, ELSEVIER, AMSTERDAM, NL, vol. 22, 4 August 2018, pages 44-49, evaluates the chemical composition of essential oil of wild mint largely used in traditional medicine e.g. in diabetes. Trans-piperitone oxide is one of the major compounds. Assad Rezwana et al, BOTANICAL REVIEW, NEW YORK BOTANICAL GARDEN, BRONX, NY, US, vol. 83, no. 4, 26 October 2017, pages 382-436, reviews the high nutritional value of Amaranthus seeds and its effect in protecting against several disorders such as diabetes, digestion disorder, heart diseases, hypertension, and others.

Among the major active ingredients of natural origin used to control cholesterol in nutraceuticals is fermented red rice, standardized in monacolin K, acting as a hydroxymethylglutaryl-CoA (HMG-CoA) inhibitor. This product proved to be really effective; however, recently, the European legislator has limited its use by reducing the maximum intake dose of monacolin K from 10 mg to values lower than 3 mg. Hence, the professionals in the sector have been pushed to search for and study possible natural alternatives.

Genetic, epidemiological and pharmacological data have led to the conclusion that by antagonizing or inhibiting Proprotein Convertase Subtilisin/Kexin Type 9 (PCSK9) cardiovascular events are reduced. This clinical outcome is mainly related to the pivotal role of PCSK9 in controlling cholesterol levels, in particular of low-density lipoproteins (LDLs).

In nature, there are several plants containing substances having a PCSK9-inhibitory effect, including berberine.

### Prior art problem

Oil extracts and essential oils, including that of Mint, have limits of use, in particular they are hydrophobic, unstable, volatile, and contain potentially toxic substances. In particular, volatile substances tend to disperse.

A strategy for overcoming these limitations and protecting essential oils normally consists in the encapsulation in carrier systems that allow to improve their physico-chemical stability, while reducing their volatility. The most common carrier systems are micro- and nano-emulsions, liposomes and lipid nanoparticles, which are all systems in liquid or semi-solid form.

On the market, some nutrients or agri-food residues are known, such as rinds and peels, unripe or damaged fruits, seeds, peels, exhausted pulps, which can serve as carriers of oil extracts and essential oils.

It is also true, however, that agri-food residues or food products differ from each other, for example in terms of nutrients (polysaccharides, lipids, and fats), or depending on the physico-chemical nature thereof and the technological processes they may undergo. The same applies, on the other hand, to oil extracts or essential oils to be carried.

In this sense, the Applicant believes that it is not always easy to select at least one carrier or carrier agent that is suitable for promoting the physico-chemical stability of one or more extracts and active principles thereof within compositions, in particular compositions in solid form.

Oil extracts or essential oils are notorious for being physically and/or chemically unstable. They can be *physically* unstable because, as in most cases, the extracts possess substances or associations of substances which are volatile in nature and easily disperse. On the other hand, they can be *chemically* unstable because, as in some cases, they contain substances having more or less easily labile, even though covalent, bonds.

For example, piperitone oxide, being characterized by an epoxy monocyclic terpene structure (shown below), is particularly reactive in the presence of amines, which are normally found in the intestinal lumen and in foods.

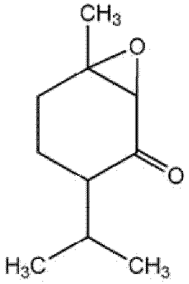

Biogenic amines are nitrogen compounds mostly produced by microbial decarboxylation of amino acids. Since micro-organisms are commonly present in the environment, as well as in the intestinal lumen, biogenic amines can be contained in foods and beverages. Their concentration is higher in quickly perishable foods, especially when fermented and rich in particular amino acids, such as fish, meat, cured meats, fruit juices, wine, cocoa, dairy products, and cheese.

In this sense, there exists the need to find a composition suitable for chemically and physically stabilizing piperitone oxide, in particular for protecting the same from the amines present in the environment, in foods and in the intestinal lumen.

In addition to the above-mentioned need, there exists also the need to find compositions/formulations, in solid form, which are able to amplify the effect of reducing abnormal levels of LDL cholesterol in the blood, preferably for treating the metabolic syndrome.

### SUMMARY OF THE INVENTION

The Applicant developed a powder composition comprising
a Mint oil extract,
an active and carrier agent consisting of Amaranth seeds flour.

This powder composition is suitable for preventing and treating mild forms of metabolic syndrome, especially for treating mild forms of hypercholesterolemia.

Finally, the Applicant developed an oral solid formulation comprising, as sole active principle, the powder composition alone, or the composition can be associated with at least another active principle, preferably serving the same use for which the composition of the invention is used, in both cases in combination with suitable excipients and/or diluents.

### Advantages of the invention

The composition in solid form for use according to the present invention developed by the Applicant has, on the one hand, technological advantages related to the stability aspects of the actives, and, on the other hand, advantages in terms of nutraceutical use.

Specifically, with the composition of the invention it is possible to simultaneously achieve:
- an increase in the chemical and physical stability of the Mint oil extract and the piperitone oxide contained therein. Such an effect is due to the presence of the carrier agent or carrier consisting of the Amaranth seeds flour; indeed, the carrier in question is able to protect the Mint oil extract and its active principle piperitone oxide; this can lead to an increase in the bioavailability and/or bioaccessibility of the Mint oil extract.

Thereby, the passage of the aforementioned extract through the intestinal epithelium can be promoted, increasing its absorption at the intestinal level;
- an effectiveness of use, mainly in the treatment of pre-pathologic conditions, such as the metabolic syndrome, since the effect deriving from the activity of the Amaranth seeds flour and that deriving from the activity of the Mint oil extract are combined. It should also be noted that an increase in the stability of the Mint oil extract results in an increase in the effectiveness thereof. In this sense, an amplified or synergistic effect of reducing abnormal levels of LDL cholesterol in the blood can be achieved.

### DESCRIPTION OF THE DRAWINGS

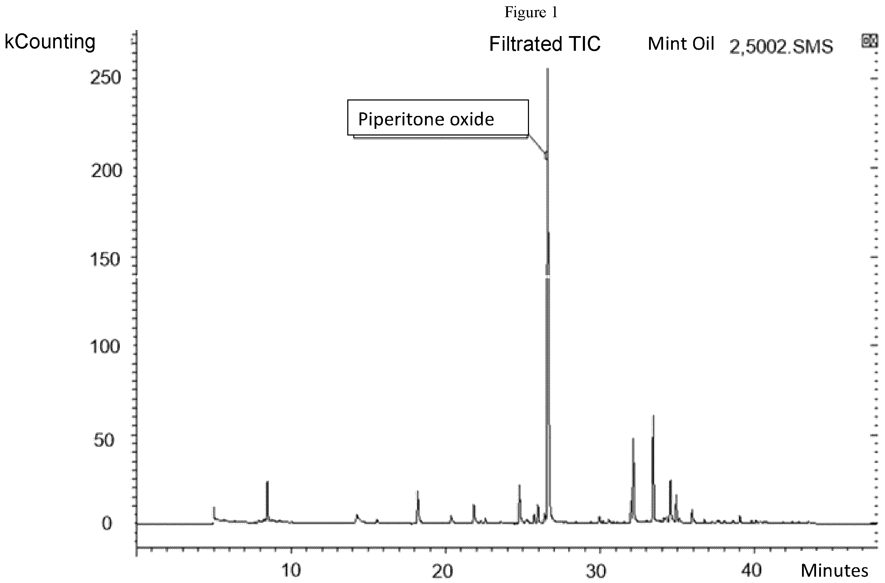

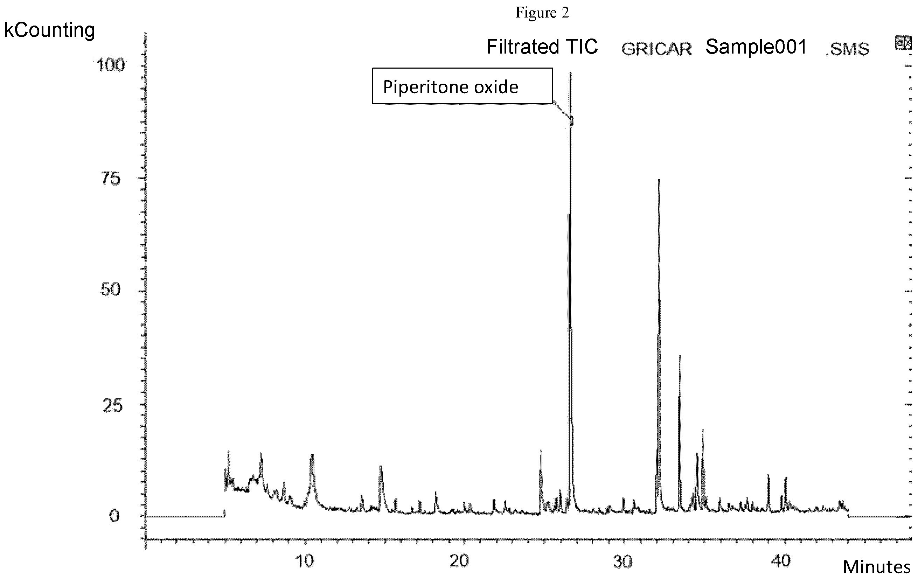

*Figs. 1* and *2* - Results for Sample No. 1 (Figure 1) and Sample No. 2 (Figure 2) of Example 4 relating to the analytical test carried out by means of a Varian 3800 gas chromatograph, equipped with a Saturn 2100 mass detector.

### DETAILED DESCRIPTION OF THE INVENTION

Hereinafter, the Applicant will describe the invention in further detail.

For the purposes of the present invention, the term "comprising" does not exclude the presence of further components which are not explicitly mentioned after this definition, while the term "consisting of" excludes this possibility.

It should be noted that, hereinafter, the Mint oil extract is also referred to by the terms "mint oil" or "mint extract", both having the same meaning as the expression "Mint oil extract".

### Composition

The composition according to the invention is in solid form ("solid composition") and comprises or consists of
a Mint oil extract, comprising piperitone oxide in an amount preferably comprised between 3% and 80%, preferably between 5% and 50% by weight, preferably of 5% by weight, based on the total weight of the extract,
an active and carrier agent consisting of Amaranth seeds flour.

The Applicant points out that the Mint oil extract and the Amaranth seeds flour are two active principles or agents.

Besides being an active, the Amaranth seeds flour is also a carrier agent or carrier.

Carrier agent or carrier means a substance or compound able to promote the chemical and/or physical stability of another substance or compound. In this case, the Amaranth seeds flour constitutes the carrier for the active Mint oil extract. Preferably, the amaranth seeds flour is a stabilizing agent or protective agent or sequestering agent for the Mint oil extract, as it is able to reduce or eliminate: on the one hand, the phenomenon of dispersion of the volatile active principles contained therein, providing physical stability; on the other hand, to reduce or eliminate the phenomenon of molecular degradation of piperitone oxide, especially of the epoxy structure thereof, ensuring chemical stability.

A further advantage of the composition constituting the object of the invention lies in the fact that, despite containing an oily liquid substance, it is in the form of powder, preferably of compressible powder, hence it is easily processable in solid forms, such as tablets, water-dispersible sachets, and not only in liquid-type oral formulations, such as micro- and nano-emulsions, soft capsules etc., liposomes and lipid nanoparticles.

According to a preferred embodiment, the composition of the invention is neither in micronized nor in nanoemulsion form; furthermore, it does not contain liposomes.

### Mint oil extract

For the purposes of the present invention, extracts generally mean products of natural origin obtained from different types of plants and medicinal herbs. Various parts of the plant are useful in obtaining these products: leaves, flowers, fruits (pulp and/or peel), seeds, roots, rhizomes, bark, etc. They can be preparations in liquid, solid form (dry extracts) or of intermediate consistency (soft extracts), obtained by suitable extraction processes; the latter include the use of appropriate solvents, the supercritical extraction, the use of maceration or percolation, or other suitable processes.

Preferably, the Mint oil extract is in an amount comprised between 1% and 90% by weight, preferably between 1% and 80% by weight, preferably between 5% and 80% by weight, preferably between 10% and 80% by weight, preferably between 20% and 80% by weight, preferably between 20% and 60% by weight, preferably of 20% by weight, 49% by weight or 60% by weight, based on the total weight of the composition.

Preferably, the Mint belongs to a species and/or subspecies and/or variants and/or basionyms selected from the group consisting of: *Mentha aquatica L., Mentha arvensis L., Mentha spicata L.* (or wild Mint), *Mentha piperita L.,* and combinations thereof, preferably is wild Mint or *Mentha spicata,* preferably of the subspecies *subsp longifolia L..*

Preferably, the piperitone oxide, which is an epoxy monocyclic terpene contained in the mint extract, is in an amount comprised between 3% and 80% by weight, preferably between 5% and 50%, preferably between 5% and 20%, preferably between 5% and 10%, preferably between 4% and 6%, preferably of 5% by weight, based on the total weight of the extract.

The Mint oil extract, highly titrated in piperitone oxide, shows two mechanisms of action useful in promoting the reduction of cholesterol levels in the blood: one is related to the reduction of the expression of LDLR, the LDL receptor, which is responsible for the transport of blood cholesterol from the liver to the body cells; the other is connected to the inhibition of the expression of PCSK9 protein.

This second mechanism is attributed precisely to piperitone oxide, a terpene which is *insoluble* in aqueous solvents (water solubility: 1.96 g/L), so that it is assumed that its *in vivo* bioavailability is not high.

It should also be noted that the Mint oil extract contains substances or actives, such as piperitone oxide, which are naturally insoluble in aqueous solvents.

The Mint oil extract is a liquid extract or essential oil. Preferably, it is obtained by supercritical extraction, a well-known extraction technique, whereby the matter to be extracted is subjected to certain temperature and pressure conditions in the presence of a gas, generally carbon dioxide CO₂ *(*S.Sut, I. Ferrarese, M. G.Lupo , N. De Zordi , E. Tripicchio, N. Ferri and S. Dall' Acqua. The Modulation of PCSK9 and LDLR by Supercritical CO2 Extracts of Mentha longifolia and Isolated Piperitone Oxide, an In Vitro Study. Molecules 2021, 26, 3886*).*

### Amaranth seeds flour

The composition preferably comprises the Amaranth seeds flour in an amount comprised between 1% and 99% by weight, preferably between 5% and 95% by weight, preferably between 5% and 90% by weight, preferably between 10% and 90% by weight, preferably between 10% and 80% by weight, preferably between 20% and 80% by weight, preferably between 20% and 70% by weight, preferably of 20% by weight or 49% by weight, or 70% by weight, based on the total weight of the composition.

Preferably, the Amaranth seeds flour belongs to a species and/or subspecies and/or variants and/or basionyms selected from the group consisting of: *Amaranthus caudatus L., Amaranthus cruentus L., Amaranthus hypochondriacus L.,* and combinations thereof, preferably is *Amaranthus caudatus L..*

The Amaranth seeds flour possesses useful properties in cholesterol metabolism (Ministry of Health on the physiological functions of botanicals in Anx. 1 of the Decree of the Ministry of Health *"Disciplina dell'impiego negli integratori alimentari di sostanze e preparati vegetali"* ("Regulations on the use of plant substances and preparations in food supplements"), August 10th, 2018, and subsequent updates). As such, it constitutes an active principle promoting cholesterol metabolism.

Preferably, the Amaranth seeds flour contains an amount of proteins comprised between 12 and 17 grams (g), preferably comprised between 13 and 16 g, preferably between 12 and 15 g, preferably of 14 g.

Preferably, the Amaranth seeds flour contains an amount of total fats comprised between 5 and 9 g, preferably comprised between 5 and 8 g, preferably between 4 and 7 g, preferably of 7 g. In particular, the amount of *Saturated Fatty Acids* (SFA) is of 1.5 g.

Preferably, the Amaranth seeds flour contains an amount of carbohydrates preferably comprised between 55 and 75 g, preferably comprised between 60 and 70 g, preferably of 65 g. In particular, the amount of sugars is of 1.7 g.

Preferably, the Amaranth seeds flour contains an amount of dietary fibers comprised between 5 and 9 g, preferably comprised between 6 and 8 g, preferably of 7 g.

It should be noted that the Amaranth seeds flour contains a relatively high proportion of polysaccharides (carbohydrates) and proteins. This makes it represent the carrier or carrier agent of choice for the Mint oil extract of the present invention.

The composition of the invention improves the stability of the Mint oil extract and also promote the dispersion thereof in the amaranth flour. In particular, it is the amaranth flour that has a sequestering, hence protective, action with respect to the piperitone oxide contained in the mint extract. Besides that, the Amaranth seeds flour is also possibly able to promote the stabilization and the dispersion of other actives, which are insoluble in aqueous solvents and contained in the Mint oil extract itself.

This phenomenon constitutes the premise of the stability of piperitone oxide in the digestive tract and, accordingly, of a greater effectiveness of the compound in controlling cholesterol levels due to two factors:
- the maximum beneficial effect of mint oil and of the active principle thereof in inhibiting PCSK9 protein expression (the PCSK9 inhibitors significantly reduce LDL cholesterol levels);
- the additional beneficial effect of amaranth seeds flour on cholesterol levels.

Preferably, the weight ratio of the Mint oil extract to the Amaranth seeds flour (amount of Mint oil extract: amount of Amaranth seeds flour) is comprised between 1:0.25 and 1:5, preferably between 1:0.3 and 1:4, preferably between 1:0.3 and 1:3.5, preferably between 1:0.5 and 1:3.5, preferably between 1:1 and 1:3.5, preferably is of 1:0.3, alternatively of 1:1 or 1:3.5, or 1:3.

It should be noted that this weight ratio is functional for the Amaranth seeds flour be able to act as a carrier and promote the stability of the Mint oil extract within the composition of the invention.

### Anti-agglomerating food additive

Preferably, the powder composition constituting the object of the present invention comprises further at least one anti-agglomerating food additive.

Preferably, the at least one anti-agglomerating food additive is selected from the group consisting of: silicon dioxide, stearic acid fatty acid salts, talc, cellulose, microcrystalline cellulose, and other cellulose derivatives, silicon-derived powders, calcium phosphates, and combinations thereof.

Preferably, the amount of the at least one anti-agglomerating food additive is comprised between 1% and 50% by weight, preferably comprised between 1% and 40% by weight, preferably comprised between 2% and 40% by weight, preferably comprised between 2% and 30% by weight, preferably is of 20% by weight, or 10% by weight, or 2% by weight, based on the total weight of the composition.

Anti-agglomerating agents are useful to prevent the formation of agglomerates or the aggregation of matter, e.g. in powder ingredients (agglomeration). These aggregation or agglomeration phenomena may be due, for example, to the absorption of water vapour contained in the atmosphere, or to the presence of fatty matter molecules that tend to bond to each other.

### Preparation process

The preparation process of the composition in solid form comprises the following steps:
i) incorporating the Mint oil extract into the Amaranth seeds flour and mixing, preferably for a time comprised between 15 and 45 minutes, preferably between 15 and 30 minutes, preferably of 15 or 30 minutes;
ii) optionally, adding at least one anti-agglomerating food additive and mixing, preferably for a time comprised between 15 and 30 minutes.

It should be noted that this process allows a tight aggregation between mint oil and amaranth flour, which performs a sequestering activity, protecting the mint oil and piperitone oxide; furthermore, it allows the incorporation thereof into solid formulations, such as tablets, sachets, single-dose powder.

### Formulation

The solid powder composition of the invention for use according to the present invention is included in an oral solid formulation comprising the same as active principle alone, or associated with at least one active ingredient for the same use, in combination with appropriate excipients and/or diluents.

According to *a first* preferred embodiment, the oral solid formulation contains:
the powder composition, as described above, as *sole* active principle,
in combination with suitable or appropriate excipients and/or diluents.

As an alternative to this first embodiment, a *second* preferred embodiment provides an oral solid formulation containing
the powder composition, as hitherto described, as active principle
at least another active ingredient or principle, preferably the latter serving the same use for which the powder composition of the invention is used,
in combination with appropriate excipients and/or diluents.

For example, at least one active ingredient or principle is an active ingredient which is antioxidant and/or hypocholesterolemic and/or vitamin-based and/or in mineral form; preferably, the at least one active ingredient, which is antioxidant and/or hypocholesterolemic and/or vitamin-based and/or in mineral form, is selected from the group consisting of: Naringin, Hesperidin, berberine, fermented red rice, bergamot extracts, betaglucans, phytosterols/phytostanols, pectins, gamma Oryzanol, policosanols, coenzyme Q10, resveratrol, plant extracts, flavonoids, vitamins, preferably Folic acid, Nicotinamide, Pyridoxine hydrochloride, Vitamin B12, minerals, and combinations thereof.

Preferably, the amount of the powder composition of the invention within the solid formulation is comprised between 20% and 45% by weight, more preferably between 25% and 40% by weight, still more preferably between 30% and 35% by weight.

It should be noted that the amount of the powder composition of the invention within the solid formulation is such regardless of whether other actives are present or not.

Preferably, the solid formulation can be in the form of tablet, capsule, powder or orodispersible or water-dispersible granulate, stick, comprising the formulation in powder or granulate form therein.

Preferably, suitable excipients and/or diluents for solid-type formulations are known to the person skilled in the art; for example, these can be selected from the group consisting of: bulking agents, thickeners, glidants, exfoliating agents, lubricants, slip agents, disintegrants, flavourings, pH adjusters, sweeteners, gelatine, plasticizers, solvents, silicon derivatives, and combinations thereof.

For example, suitable excipients and/or diluents included in the solid-type formulation are selected from the group consisting of: microcrystalline cellulose, modified cellulose derivatives (e.g., hydroxypropyl cellulose), dibasic calcium phosphate, silicon dioxide, silicon-derived powders, magnesium stearate, and mixtures thereof.

Preferably, the solid formulation is in the form of nutraceutical (or nutraceutical formulation), or in the form of food supplement, or still in the form of Food for Special Medical Purposes (FSMP).

Nutraceutical means a food derivative to which, besides the basic nutritive value, the capability of "optimizing" physiological functions is attributed.

Food supplement means a formulation falling under the definition encompassed by the Directive 2002/46/CE and the subsequent amendments. In this legislation, food supplements are defined as: "dietary products designed to integrate the normal diet that are a concentrated source of nutrients, such as vitamins and minerals, or of other substances with nutritional or physiological effects, especially but not only amino acids, essential fatty acids, fibres and plant-based extracts, either mono- or multi-compounds, in pre-dosed forms".

Foods for Special Medical Purposes (FSMPs) mean food products specifically processed or formulated for the dietary management of patients with specific nutritional needs, including infants, to be used "under medical supervision". FSMPs are intended for the exclusive or partial feeding of patients having a limited or altered ability to take, digest, absorb, metabolize or eliminate ordinary foods or certain nutrients contained therein, or having specific nutritional needs caused by clinical conditions. FSMPs differ from other foodstuffs and food supplements themselves due to some characteristics, such as: they require medical supervision; they can replace - totally or partially - the normal diet; they are indicated for specific pathological conditions. As for the composition, FSMPs are regulated by the Regulation 609/13.

### Usage

The composition of the invention is for use in preventing and/or treating the mild metabolic syndrome. Preferably, the solid composition of the invention is for use in reducing LDL cholesterol levels.

Metabolic syndrome means a combination of diabetes, high blood pressure, and obesity. These are three individual cardiovascular risk factors; when they are present simultaneously, cardiovascular risk is known to increase compared to the individual conditions.

The oral solid formulation according to the invention is for use in preventing and/or treating mild metabolic syndrome, in particular in treating and preventing mild hypercholesterolemia. Preferably, the solid formulation is for use in reducing LDL cholesterol levels.

### EXAMPLES

Below, the Applicant provides examples for merely illustrative, but non-limiting purposes.

### Example 1 - Composition in solid form

| **Ingredient** | **%** |
|---|---|
| Supercritical oil extract of *Mentha longifolia,* titrated to 5% in piperitone oxide | 60 |
| *Amaranthus caudatus L.* seeds flour | 20 |
| Additives | 20 |

### Preparation method of the composition according to Example 1

1. Incorporating the Mint oil extract into the Amaranth seeds flour and mixing using a planetary kneader or a whisk or ribbon mixer for a processing time of 30 minutes;
2. adding suitable anti-agglomerating food additives and incorporating by mixing with the same equipment for further 30 minutes.

### Example 2 - Composition in solid form

| **Ingredient** | **%** |
|---|---|
| Supercritical oil extract of *Mentha longifolia,* titrated to 5% in piperitone oxide | 20 |
| *Amaranthus caudatus L.* seeds flour | 70 |
| Additives | 10 |

### Preparation method of the composition according to Example 2

1. Incorporating the Mint oil extract into the Amaranth seeds flour using a planetary kneader or a whisk or ribbon mixer for a processing time of 15 minutes;
2. adding the additives and incorporating by mixing with the same equipment for further 30 minutes.

### Example 3 - Composition in solid form

| **Ingredient** | **%** |
|---|---|
| Supercritical oil extract of *Mentha longifolia,* titrated to 5% in piperitone oxide | 49 |
| *Amaranthus caudatus L.* seeds flour | 49 |
| Additives | 2 |

### Preparation method of the composition according to Example 3

1. Incorporating the oil or Mint oil extract into the Amaranth seeds flour using a planetary kneader or a whisk or ribbon mixer for a processing time of 30 minutes;
2. adding the additives and incorporating by mixing with the same equipment for further 2 minutes.

### Example 4 - Experimental test to prove the sequestering effect of amaranth flour on mint oil

### Preparation of Sample No. 1 (Comparative sample)

2.5 g of mint oil (very dark green in colour) were added to 100 ml of demineralized water, placed under stirring for 10 minutes, centrifuged at 4000 rpm for 5 minutes, and filtered with filter paper in a transparent beaker.

### Preparation of Sample No. 2 (Sample according to the invention)

10 g of a composition comprising mint oil (very dark green in colour) and amaranth seeds flour (light cream-beige in colour), containing 25% of mint oil (corresponding to 2.5 g of mint oil), were added to 100 ml of demineralized water, placed under stirring for 10 minutes, centrifuged at 4000 rpm for 5 minutes, and filtered with filter paper in a transparent beaker.

Analysis were performed by means of a Varian 3800 gas chromatograph, equipped with a Saturn 2100 mass detector.

The solutions were analyzed after the addition of internal standard (nonanol), normalizing the area of piperitone oxide based on the amount of internal standard.

The piperitone oxide content was analytically checked in both filtered samples and provided the following results:
Sample No. 1: Content of piperitone oxide 8.85 mg/mL (see FIG. 1).
Sample No. 2: Content of piperitone oxide 2.54 mg/ml (see FIG. 2).

The concentration of piperitone oxide in the filtrate is reduced by more than a third in sample 2 (compound of mint oil and amaranth flour carrier), compared to sample 1 (mint oil as is). This demonstrates a real sequestering and protective effect of amaranth on piperitone oxide.

### Example 5 - Formulation comprising the composition in solid form of the invention

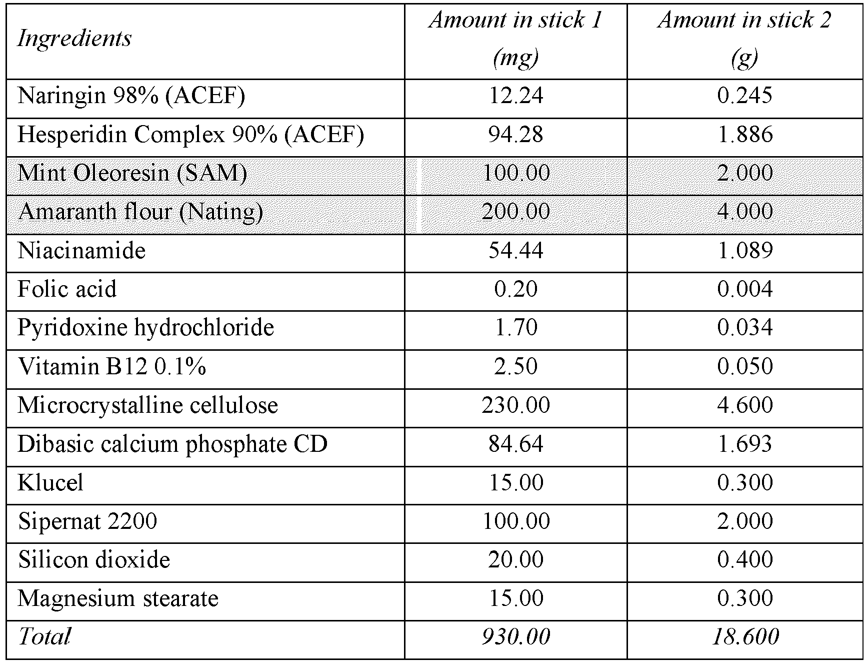

### LITERATURE

1. M.Tafrihi, M. Imran , T. Tufail , T. Aslam Gondal, G. Caruso , S. Sharma , R. Sharma, M. Atanassova , L. Atanassov, P. V. Tsouh Fokou and R. Pezzani. The Wonderful Activities of the Genus Mentha: Not Only Antioxidant Properties. Molecules 2021, 26, 1118.
2. M. Hosein Farzaei, R. Bahramsoltani, A. Ghobadi, F. Farzaei, F. Najafi. Pharmacological activity of Mentha longifolia and its phytoconstituents. J. Tradit Chin Med 2017 October 15; 37(5): 710-720.
3. S. Singha, S.S. Dasa, G. Singha, M. Perotti, C. Schuffb and C. A.N. Catalánb. In vitro antioxidant potentials and chemistry of essential oils and oleoresins from fresh and sun-dried Mentha longifolia L. Journal of Essential Oil Research, 27:1, 61-69.
4. A. Santini and E. Novellino. Nutraceuticals in hypercholesterolaemia: an overview. British Journal of Pharmacology (2017) 174 1450-1463.
5. S.Sut, I. Ferrarese, M. G.Lupo , N. De Zordi , E. Tripicchio, N. Ferri and S. Dall' Acqua. The Modulation of PCSK9 and LDLR by Supercritical CO2 Extracts of Mentha longifolia and Isolated Piperitone Oxide, an In Vitro Study. Molecules 2021, 26, 3886.
6. M. P. Adorni, F. Zimetti, M. G. Lupo, M. Ruscica and N.Ferri. Naturally Occurring PCSK9 Inhibitors. Nutrients 2020, 12, 1440.
7. Cinzia Cimino 1 , Oriana Maria Maurel 2, Teresa Musumeci 1 , Angela Bonaccorso 1 , Filippo Drago 2, Eliana Maria Barbosa Souto 3,4, Rosario Pignatello 1 and Claudia Carbone. Essential Oils: Pharmaceutical Applications and Encapsulation Strategies into Lipid-Based Delivery Systems. Pharmaceutics 2021, 13, 327.
8. Z. Dajic Stevanovic, E. Sieniawska, K. Glowniak., N Obradovic and I. Pajic-Lijakovic. Natural Macromolecules as Carriers for Essential Oils: From Extraction to Biomedical Application. Frontiers in Bioengineering and Biotechnology June 2020, Volume 8, Article 563.
9. P. R. Venskutonis and P. Kraujalis. Nutritional Components of Amaranth Seeds and Vegetables: A Review on Composition, Properties, and Uses. Institute of Food Technologists. Comprehensive Reviews in Food Science and Food Safety 2013, pag 381.
10. B. Skwarylo-Bednarz, P. M. St 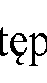 pniak, A. Jamiolkowska, M. Kopacki, A. Krzepi 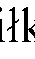 ko, H. Klikocka. Amaranth Seeds ad a Source of Nutrients and Bioactive Substances in Human Diet. Acta Sci. Pol. Hortorum Cultus, 19(6) 2020, 153-164.
11. V. M. Caselato-Sousa and J. Amaya-Farfan State of Knowledge on Amaranth Grain: A Review Journal of Food Science 2012 Vol. 77, Nr. 4.
12. Z. Chmelik, M. Vrablik. The effect of Amaranth Flour on Plasma Cholesterol Levels: another option for Cardiovascular Risk Lowering? Abstracts / Atherosclerosis 241 (2015) e149-e229.

## Claims

1. A powder composition comprising
a Mint oil extract, comprising piperitone oxide in an amount comprised between 3% and 30% by weight based on the total weight of the extract,
an active and carrier agent consisting of Amaranth seeds flour,
for use in treating and preventing mild forms of metabolic syndrome.

2. The composition for use according to claim 1, for use in preventing and treating mild hypercholesterolemia.

3. The powder composition for use according to claim 1 or 2, wherein the Mint oil extract is in an amount comprised between 1% and 90% by weight based on the total weight of the composition.

4. The powder composition for use according to any one of claims from 1 to 3, wherein the Amaranth seeds flour is in an amount comprised between 1% and 99% by weight based on the total weight of the composition.

5. The powder composition for use according to any one of claims from 1 to 4, wherein the Mint belongs to a species and/or subspecies and/or variants and/or basionyms selected from the group consisting of: *Mentha aquatica L., Mentha arvensis L., Mentha spicata L., Mentha piperita L.,* and combinations thereof.

6. The powder composition for use according to any one of claims from 1 to 5, wherein the Amaranth seeds flour belongs to a species and/or subspecies and/or variants and/or basionyms selected from the group consisting of: *Amaranthus caudatus L., Amaranthus cruentus L., Amaranthus hypochondriacus L.,* and combinations thereof.

7. The powder composition for use according to any one of claims from 1 to 6, further comprising at least one anti-agglomerating food additive.

8. The powder composition for use according to claim 7, wherein the at least one anti-agglomerating food additive is selected from the group consisting of: silicon dioxide, stearic acid fatty acid salts, talc, cellulose, microcrystalline cellulose, calcium phosphates, and combinations thereof.

9. The powder composition for use according to any one of claims from 1 to 8, in the form of:
an oral solid formulation containing
the powder composition according to any one of claims from 1 to 8, as *sole* active principle,
in combination with suitable excipients and/or diluents,
or
in the form of an oral solid formulation containing
the powder composition according to any one of claims from 1 to 8, as active principle,
at least another active principle,
in combination with appropriate excipients and/or diluents.

10. The powder composition for use according to claim 9, wherein said oral solid composition is in the form of nutraceutical, food supplement or Food for Special Medical Purposes.

## Patentansprüche

1. Eine Pulverzusammensetzung, umfassend einen Minzölextrakt, der Piperitonoxid in einer Menge zwischen 3 Gew.-% und 30 Gew.-%, bezogen auf das Gesamtgewicht des Extrakts, umfasst, ein aktives und Trägermittel, das aus Amaranthsamenmehl besteht, zur Verwendung bei der Behandlung und Vorbeugung von leichten Formen des metabolischen Syndroms.

2. Die Zusammensetzung zur Verwendung nach Anspruch 1, zur Verwendung bei der Vorbeugung und Behandlung von leichter Hypercholesterinämie.

3. Die Pulverzusammensetzung zur Verwendung nach Anspruch 1 oder 2, wobei der Minzölextrakt in einer Menge zwischen 1 Gew.-% und 90 Gew.-%, bezogen auf das Gesamtgewicht der Zusammensetzung, vorliegt.

4. Die Pulverzusammensetzung zur Verwendung nach einem der Ansprüche 1 bis 3, wobei das Amaranthsamenmehl in einer Menge zwischen 1 Gew.-% und 99 Gew.-%, bezogen auf das Gesamtgewicht der Zusammensetzung, vorliegt.

5. Die Pulverzusammensetzung zur Verwendung nach einem der Ansprüche 1 bis 4, wobei die Minze zu einer Spezies und/oder Subspezies und/oder Variante und/oder einem Basionym gehört, die/das aus der Gruppe ausgewählt ist, die aus *Mentha aquatica L., Mentha arvensis L., Mentha spicata L., Mentha piperita* L. und Kombinationen davon besteht.

6. Die Pulverzusammensetzung zur Verwendung nach einem der Ansprüche 1 bis 5, wobei das Amaranthsamenmehl zu einer Spezies und/oder Subspezies und/oder Variante und/oder einem Basionym gehört, die/das aus der Gruppe ausgewählt ist, die aus *Amaranthus caudatus L., Amaranthus cruentus L., Amaranthus hypochondriacus* L. und Kombinationen davon besteht.

7. Die Pulverzusammensetzung zur Verwendung nach einem der Ansprüche 1 bis 6, ferner umfassend mindestens ein Trennmittel als Lebensmittelzusatzstoff.

8. Die Pulverzusammensetzung zur Verwendung nach Anspruch 7, wobei das mindestens eine Trennmittel als Lebensmittelzusatzstoff aus der Gruppe ausgewählt ist, die aus Siliciumdioxid, Salzen von Fettsäuren der Stearinsäure, Talk, Cellulose, mikrokristalliner Cellulose, Calciumphosphaten und Kombinationen davon besteht.

9. Die Pulverzusammensetzung zur Verwendung nach einem der Ansprüche 1 bis 8, in Form von: einer oralen festen Formulierung, enthaltend die Pulverzusammensetzung nach einem der Ansprüche 1 bis 8 als einzigen Wirkstoff, in Kombination mit geeigneten Hilfsstoffen und/oder Verdünnungsmitteln, oder in Form einer oralen festen Formulierung, enthaltend die Pulverzusammensetzung nach einem der Ansprüche 1 bis 8 als Wirkstoff, mindestens einen weiteren Wirkstoff, in Kombination mit geeigneten Hilfsstoffen und/oder Verdünnungsmitteln.

10. Die Pulverzusammensetzung zur Verwendung nach Anspruch 9, wobei die orale feste Zusammensetzung in Form eines Nutrazeutikums, eines Nahrungsergänzungsmittels oder eines Lebensmittels für besondere medizinische Zwecke vorliegt.

## Revendications

1. Une composition en poudre comprenant un extrait d'huile de Menthe, comprenant de l'oxyde de pipéritone en une quantité comprise entre 3 % et 30 % en poids par rapport au poids total de l'extrait, un agent actif et vecteur consistant en de la farine de graines d'Amarante, pour son utilisation dans le traitement et la prévention de formes légères du syndrome métabolique.

2. La composition pour son utilisation selon la revendication 1, pour son utilisation dans la prévention et le traitement de l'hypercholestérolémie légère.

3. La composition en poudre pour son utilisation selon la revendication 1 ou 2, dans laquelle ledit extrait d'huile de Menthe est en une quantité comprise entre 1 % et 90 % en poids par rapport au poids total de la composition.

4. La composition en poudre pour son utilisation selon l'une quelconque des revendications 1 à 3, dans laquelle ladite farine de graines d'Amarante est en une quantité comprise entre 1 % et 99 % en poids par rapport au poids total de la composition.

5. La composition en poudre pour son utilisation selon l'une quelconque des revendications 1 à 4, dans laquelle la Menthe appartient à une espèce et/ou sous-espèce et/ou variante et/ou un basionyme choisi dans le groupe consistant en: *Mentha aquatica L., Mentha arvensis L., Mentha spicata L., Mentha piperita* L., et des combinaisons de celles-ci.

6. La composition en poudre pour son utilisation selon l'une quelconque des revendications 1 à 5, dans laquelle la farine de graines d'Amarante provient d'une espèce et/ou sous-espèce et/ou variante et/ou un basionyme choisi dans le groupe consistant en *: Amaranthus caudatus L., Amaranthus cruentus L., Amaranthus hypochondriacus* L., et des combinaisons de celles-ci.

7. La composition en poudre pour son utilisation selon l'une quelconque des revendications 1 à 6, comprenant en outre au moins un additif alimentaire anti-agglomérant.

8. La composition en poudre pour son utilisation selon la revendication 7, dans laquelle ledit au moins un additif alimentaire anti-agglomérant est choisi dans le groupe consistant en : du dioxyde de silicium, des sels de l'acide gras stéarique, du talc, de la cellulose, de la cellulose microcristalline, des phosphates de calcium, et des combinaisons de ceux-ci.

9. La composition en poudre pour son utilisation selon l'une quelconque des revendications 1 à 8, sous la forme de : une formulation solide orale contenant la composition en poudre selon l'une quelconque des revendications 1 à 8, en tant que principe actif unique, en combinaison avec des excipients et/ou diluants appropriés, ou sous la forme d'une formulation solide orale contenant la composition en poudre selon l'une quelconque des revendications 1 à 8, en tant que principe actif, au moins un autre principe actif, en combinaison avec des excipients et/ou diluants appropriés.

10. La composition en poudre pour son utilisation selon la revendication 9, dans laquelle ladite formulation solide orale est sous la forme d'un nutraceutique, d'un complément alimentaire ou d'un aliment destiné à des fins médicales spéciales.
